# EUROPÄISCHE PATENTSCHRIFT

(11) **EP 1 428 827 B1**
(45) Veröffentlichungstag und Bekanntmachung des Hinweises auf die Patenterteilung: **28.03.2012**
(21) Anmeldenummer: 03027357.7
(22) Anmeldetag: 27.11.2003
(51) Int. Cl.: C07D 519/00, C07D 495/04, C08G 61/12

(54) **Derivate von 2,2'-Di (3,4-alkylendioxythiophen) deren Herstellung und Verwendung**
Derivatives of 2,2'-Di (3,4-alkylenedioxythiophen) their preparation and use
Dérivés de 2,2'-Di (3,4-alkylenedioxythiophène), leur préparation et utilisation

(30) Priorität: 10.12.2002 DE 10257539
(43) Veröffentlichungstag der Anmeldung: 16.06.2004
(60) Teilanmeldung: 11000310.0
(73) Patentinhaber: Heraeus Precious Metals GmbH & Co. KG, 63450 Hanau (DE)
(72) Erfinder: Reuter, Knud, Dr., 47800 Krefeld (DE)
(74) Vertreter: Herzog, Martin

(56) Entgegenhaltungen:
- EP-A- 0 339 340
- EP-A- 1 289 031
- DATABASE CA [Online] CHEMICAL ABSTRACTS SERVICE, COLUMBUS, OHIO, US; ZOTTI, GIANNI ET AL: "Electrochemical and chemical synthesis and characterization of sulfonated poly(3,4-ethylenedioxythiophene): a novel water-soluble and highly conductive conjugated oligomer" retrieved from STN Database accession no. 138:107475 XP002267046 & MACROMOLECULAR CHEMISTRY AND PHYSICS (2002), 203(13), 1958-1964 ,
- ZOTTI ET AL: "Mono- and multilayers of oligoethylene oxide-modified poly(3,4-ethylenedioxythiophene) on ITO and glass surfaces" CHEM. MATER., Bd. 15, 20. Mai 2003 (2003-05-20), Seiten 2222-28, XP002267044
- AKOUDAD ET AL: "Electrochemical Synthesis of Poly(3,4-thylenedioxythiophene) from a Dimer Precursor" SYNTHETIC METALS, Bd. 93, Nr. 2, 1998, Seiten 111-114, XP002267045
- SOTZING G A ET AL: "POLY(3,4-ETHYLENEDIOXYTHIOPHENE) (PEDOT) PREPARED VIA ELECTROCHEMICAL POLYMERIZATION OF EDOT, 2,2'-BIS (3,4'-ETHYLENEDIOXYTHIOPHENE) (BIEDOT), AND THEIR TMS DERIVATIVES" ADVANCED MATERIALS, VCH VERLAGSGESELLSCHAFT, WEINHEIM, DE, Bd. 9, Nr. 10, 8. August 1997 (1997-08-08), Seiten 795-798, XP000695455 ISSN: 0935-9648
- PATENT ABSTRACTS OF JAPAN vol. 2000, no. 07, 29. September 2000 (2000-09-29) & JP 2000 106223 A (FUJI PHOTO FILM CO LTD), 11. April 2000 (2000-04-11)
- CAO, JIE ET AL: "Synthesis and Characterization of Bis(3,4-ethylene-dioxythiophene)- (4,4'-dialkyl-2,2'-bithiazole) Co-oligomers for Electronic Applications" CHEMISTRY OF MATERIALS (2003), 15(2), 404-411 , XP002267109

## Beschreibung

Die Erfindung betrifft ein Verfahren zur Herstellung von 2,2'-Di(alkylen-dioxythiophen)en (in der Literatur auch 2,2'-Bis(3,4-alklendioxythiophen)e oder 3,4-(Alkylendioxy)-2.2'-bithiophene genannt), sowie deren

Die Verbindundsklasse der π-konjugierten Polymere war in den letzten Jahrzehnten Gegenstand zahlreicher Veröffentlichungen. Sie werden auch als leitfähige Polymere oder als synthetische. Metalle bezeichnet.

Wegen der erheblichen Delokalisierung der π-Elektronen entlang der Hauptkette zeigen diese Polymere interessante (nichtlineare) optische Eigenschaften, und nach Oxidation oder Reduktion stellen sie gute elektrische Leiter dar. Dadurch können diese Verbindungen in verschiedenen praktischen Anwendungsgebieten eingesetzt werden, wie z.B. in der Datenspeicherung, der optischen Signalverarbeitung, der Unterdrückung elektromagnetischer Störungen (EMI) und der Sonnenenergieumwandlung, sowie iD wiederaufladbaren Batterien, Licht emittierenden Dioden (LED's), Feldeffekttransistoren. Leiterplatten, Sensoren, Kondensatoren und anti-statischen Materialien.

Beispiele für bekannte π-konjugierte Polymere sind Polypyrrole, Polythiophene, Polyaniline. Polyacerylene, Polyphenylene und Poly(p-phenylen-vinylene).

Ein besonders wichtiges und technisch genutztes Polythiophen ist das Poly(3,4-ethylendioxythiophen), das in seiner kationischen Form sehr hohe Leitfähigkeiten aufweist. Weiterhin sind eine Reibe von Polymeren bekannt, die den 3,4-Ethylendioxythiophen-Baustein enthalten und wegen ihrer Elekirolumineszenz als Rohstoffe für Licht emittierende Dioden geeignet sind. Dabei ist es zur Einstellung der Emissions-Wellenlängen und der Lumineszenz-Intensität besonders wertvoll, auch auf 2,2'-Di(3,4-ethylendioxythiophen)-Bausteine zurückgreifen zu können. (Lit. z. B. A. Donat-Bouillud, I. Lévesque, Y. Tao, M. D'Iorio, S. Beaupré, P. Blondin, M. Ranger, J. Bouchard und M. Leclerc, Chem. Mater. 2000, 12, S. 1931-1936).

Problematisch für diesen Einsatz ist, dass die benötigten 2,2'-Di(3,4-ethylendioxythiophen)e bisher nur über aufwändige metallorganische Synthesen zugänglich sind, die - je nach Reaktionsbedingungen - zu geringen Ausbeuten und/oder zu nur mäßigen Reinheiten führen können.

Die 2,2'-Di(3,4-ethylendioxythiophen)e werden in der Literatur bisher in der Regel nach folgendem Verfahren (Ullmann-Kupplung) hergestellt:

3,4-Ethylendioxythiophen wird mittels n-Butyllithium bei -78°C in absolutem Tetrahydrofuran unter Schutzgas lithiiert und anschließend mit Kupfer-II-chlorid oxidativ zum 2,2'-Di(3,4-ethylendioxythiophen) gekuppelt.

Die im Folgenden aufgeführten Originalarbeiten illustrieren die Nachteile dieses Syntheseweges, insbesondere bezüglich der Reaktionsbedingungen, Ausbeuten und Reinheiten:

Aus G. A. Sotzing, J. R. Reynolds und P. J. Steel, Adv. Mater. 1997, 9(10), S. 795 - 798 geht hervor, dass nur ein unreines Produkt erhalten wurde (Schmp. 183 - 185°C). In A. Donat-Bouillud, I. Levesque, Y. Tao, M. D'Iorio, S. Beaupre, P. Blondin, M. Ranger, J. Bouchard und M. Leclerc, Chem. Mater. 2000, 12, S. 1931-1936 wird zwar nach gleichem Verfahren die Herstellung eines reineren Produktes beschrieben (Schmp. 203°C), jedoch beträgt die Ausbeute nur 27,7 % der Theorie. Durch eine Verlängerung der Reaktionszeit für die Ullmann-Kupplung wurde von A. K. Mohanakrishnan, A. Hucke, M. A. Lyon, M. V. Lakshmikantham und M. P. Cava (Tetrahedron 55 (1999), S. 11745 - 11754) eine Ausbeute von 84 % eines relativ reinen Produktes erhalten (Schmp. 203 - 204°C). Hierfür musste die Reaktionszeit jedoch versechsfacht werden.

Wesentliche Nachteile des in allen diesen Publikationen benutzten Verfahrens zur Herstellung von 2,2'-Di(3,4-ethylendioxythiophen) sind: 1) Für die Lithiierung muss bei tiefen Temperaturen (- 78°C) mit Hilfe einer externen Kältemischung, zum Beispiel aus Aceton/Trockeneis, gearbeitet werden. 2) n-Butyllithium als metallorganisches Reagens ist kostspielig und in der Handhabung luft- und feuchtigkeitsempfindlich sowie, insbesondere bei Hinzutritt von Feuchtigkeit, leicht entzündlich. 3) Die gesamte Reaktion muss wegen der Empfindlichkeit des Butyllithiums und des als Zwischenprodukt gebildeten lithiierten Thiophens unter Schutzgas (Stickstoff oder Argon) durchgeführt werden.

Die von S. S. Zhu und T. M. Swager in J. Am. Chem. Soc. 1997, 119, S. 12568 - 12577 angegebene Variante dieses Verfahrens (Lithiierung bei -10°C unter Zusatz von Tetramethylethylendiamin und oxidative Kupplung mittels Eisen-IIIacetylacetonat unter Rückfluss in THF) ergibt keine Vereinfachungen, da Kältemischungen und Schutzgastechnik ebenfalls erforderlich sind. Die tatsächliche Ausbeute beträgt 50 % der Theorie (0,99 g von maximal möglichen 1,97 g); die angegebenen 99 % beruhen auf einem Druckfehler.

Ein weiterer wesentlicher Nachteil der zitierten metallorganischen Verfahrensweisen ist deren eingeschränkte Anwendbarkeit. An der Ethylen-Brücke substituierte 2,2'-Di(3,4-ethylendioxythiopben)e sind daher bisher nicht beschrieben worden. Sie wären nach der Methode der Lithiierung mit anschließender Ullmann-Reaktion nicht auf gezieltem Weg zugänglich, da bei zahlreichen denkbaren Substituenten an der Ethylen-Brücke - z. B. Hydroxyalkylgruppen, Carbonyl- oder Heterocarbonylgruppen, Doppelbindungen etc. - eine Lithiierung dieser Substituenten als Neben-oder Hauptreaktion einträte. Auch 2,2'-Di(3,4-alkylendioxythiophen)e mit anderen Alkylenbrücken als der Ethylenbrücke wurden bisher in der Literatur nicht beschrieben.

Somit besteht Bedarf nach einer neuen Methode zur Herstellung bekannter sowie neuartiger 2,2'-Di(3,4-alkylendioxythiophen)e, bei der
- auf den Einsatz von niedrigen Temperaturen, d. h. Rältemischungen oder ähnlichem zur externen Kühlung, verzichtet werden kann
- eine Durchführung ohne Schutzgastechnik an Luft möglich ist
- eine breite Anwendbarkeit, beispielsweise für die Herstellung an der Ethylen-Brücke funktionell substituierter 2,2'-Di(3,4-ethylendioxythiophen)e oder soustiger gegebenenfalls substituierter 2,2'-Di(3,4-alkylendioxythiophen)e, gegeben ist.

Diese Aufgabe wird erfindungsgemäß dadurch gelöst, dass auf einfache Weise 2,2'-Di(3,4-alhylendioxthiophen)e, im Folgenden auch als Verbindungen dcr Formel (I) bezeichnet, hergestellt werden können, indem Verbindungen der allgameinen Formel (II), 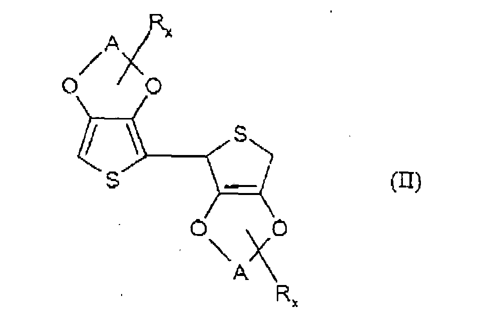 worin
- A: für einen C₂-C₄-Alkylenrest steht,
- R: für einen oder mehrere, gleiche oder verschiedene lineare(n) oder ver- zweigte(n), gegebenenfalls substituierte(n) C₁-C₁₈Alkylrest(e), gege- benenfalls substituierte(n) C₅-C₁₂-Cycloalkylrest(e), gegebenfalls substituierts(n) C₆-C₁₆-Alkyrest(e), gegebenenfalls substituierte(n) C₁- C₄-Hydroxyalkylrest(e) oder einen oder mehrere Hydroxylrest(e) steht,
- x: für eine ganze Zahl von () bis 8 steht,
mit einem Dehydrierungsmittel umgesetzt werden.

Gegenstand der vorliegenden Erfindung ist daher ein Verfahren zur Herstellung von Verbindungen der allgemeinen Formel (I), 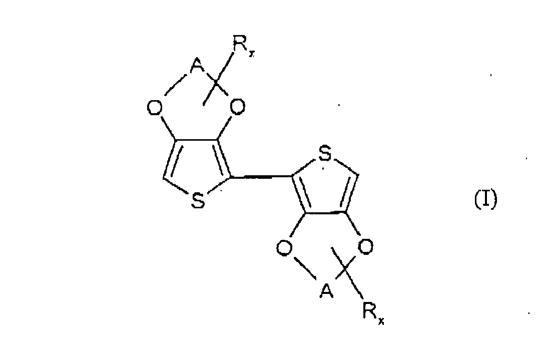 worin
- A: für einen C₁-C₄-Alkylenrest, bevorzugt für einen gegebenefalls substituierten Ethylenrest steht,
- R: für einen oder mehrere, gleiche oder verschiedene lineare(n) oder ver- zweigte(n), gegebenenfalls substituierte(n) C₁-C₁₈-Alkylrest(e), gegebenen- falls substituierte(n) C₅-C₁₂-Cycloalkylrest(e), gegebenenfalls substituierte(n) C₆-C₁₆-Aryltrest(e), gegebenenfalls substituierte(n) C₁-C₄-Hydroxyalkyl- rest(e) oder einen oder mehrere Hydroxylrest(e), bevorzugt für Methyl, Ethyl, n-Propyl, n-Butyl, n-Hexyl n-Octyl, n-Decyl, n-Dodecyl, n-Tetradecyl oder Hydroxymethyl steht,
- x: für eine ganze Zahl von 0 bis 8, bevorzugt für eine ganze Zahl von 0 bis 4, besonders bevorzugt für 0 oder 1 steht,
dadurch gekennzeichnet, dass Verbindungen der allgemeinen Formel (II), 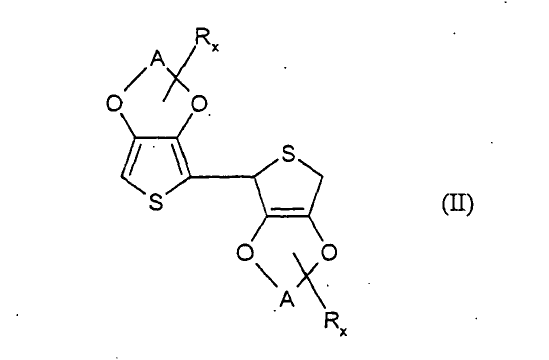 worin A, R und x die für die allgemeine Formel (I) genannte Bedeutung haben,
mit einem Dehydrierungsmittel umgesetzt werden.

Eine bevorzugte Ausführungsform des erfindungsgemäßen Verfahrens ist ein solches zur Herstellung der Verbindung der Formel (I-a) (auch als 2,2'-Di(3,4-ethylendioxythiophen) bezeichnet) - 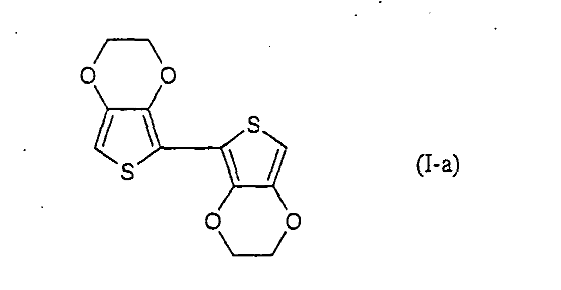 dadurch gekennzeichnet, dass Verbindungen der Formel (II-a) 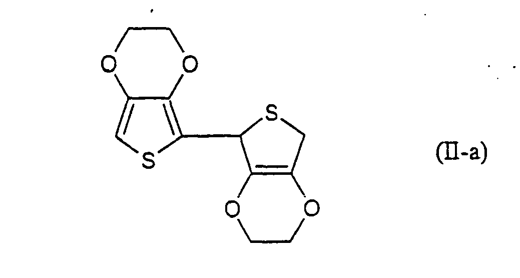 mit einem Dehydrierungsmittel umgesetzt werden.

Die Verbindungen der Formel (II) können je nach Wahl von A und x verschiedene Stereoisomeren, d.h. Enantiomeren oder Diastereoisomeren, umfassen. Im Rahmen der Erfindung sind unter Verbindungen der Formel (II) entweder die reinen Enantiomeren oder Diastereoisomeren oder Mischungen aus diesen in einem beliebigen Mischungsverhältnis zu verstehen. Beispielsweise können Verbindungen der Formel (II-a) die beiden Enantiomeren (II-a-S) oder (II-a-R) 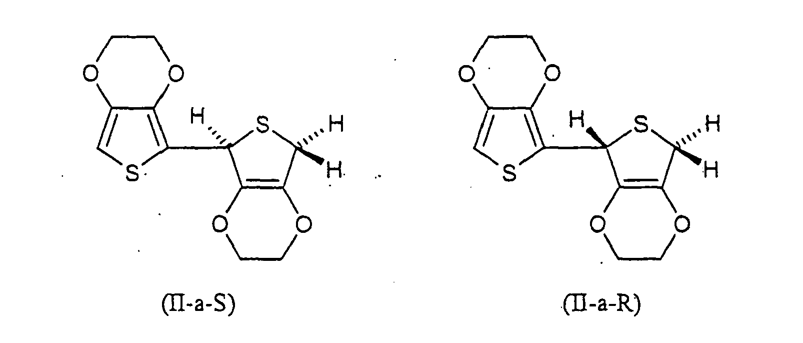 in reiner Form oder Mischungen aus diesen in beliebigen Mischungsverhältnissen von (II-a-S) zu (II-a-R) sein.

Die Verbindungen der Formel (II) können nach einem Verfahren hergestellt werden, welches in der noch nicht veröffentlichten deutschen Patentanmeldung DE 10 229 218 beschrieben ist. Dazu werden Verbindungen der Formel (VI) 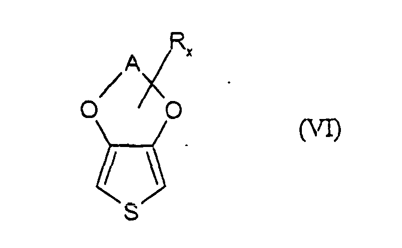 worin A, R und x die für die allgemeine Formel (I) genannte Bedeutung haben, in Gegenwart von Lewis-Säuren, bevorzugt nicht-oxidierenden Lewis-Säuren wie beispielsweise Bortrifluorid (z.B. in Form des Etherats oder eines anderen BF₃-Komplexes, z.B. mit Tetrahydrofuran), Antimonpentachlorid, Aluminiumtrichlorid, Titantetrachlorid, Zinntetrachlorid und Zinkchlorid, oder in Gegenwart von Protonensäuren, bevorzugt ausgewählt aus der Gruppe der Sulfon- oder Carbonsäuren - wie beispielsweise p-Toluolsulfonsäure, Methansulfonsäure, Campher-10-sulfonsäure oder Trifluoressigsäure - oder Supersäuren, als Katalysator miteinander umgesetzt werden. Die Verbindungen der allgemeinen Formel (VI) werden beispielsweise in einem organischen Lösungsmittel, z.B. halogenierten aliphatischen Kohlenwasserstoffen, wie Methylenchlorid oder Chloroform, Alkoholen, wie Methanol oder Ethanol, oder Aromaten, wie Toluol oder Xylol, bei Temperaturen von 0°C bis 40°C gegebenenfalls unter Schutzgas mit dem Katalysator umgesetzt. Dabei werden bevorzugt 0,01 bis 50 Gew.-% Lewissäure oder 0,5 bis 80 Gew.-% Protonensäure als Katalysator eingesetzt (Gew.% bezogen auf das zu dimerisierende Thiophenmonomer). Die Umsetzung kann mehrere Stunden durchgeführt oder ¹H-NMR-spektroskopisch verfolgt und zum geeigneten Zeitpunkt durch Zugabe von Basen (z.B. wässrige Na₂CO₃-Lösung) oder Alkoholen (z.B. Ethanol) abgebrochen werden. Die erhaltenen Verbindungen der allgemeinen Formel (II) können säulenchromatographisch aufgereinigt werden.

C₂-C₄-Alkylenreste A sind im Rahmen der Erfindung Ethylen, n-Propylen oder n-Butylen. Lineare oder verzweigte C₁-C₁₈-Alkylreste sind im Rahmen der Erfindung beispielsweise Methyl, Ethyl, n- oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-.Dimethylpropyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Hexadecyl oder n-Octadecyl; C₅-C₁₂-Cycloalkylreste sind beispielsweise Cyclopentryl, Cyclohexyl, Cycloheptyl, Cyclooctyl, Cyclononyl oder Cyclodecyl, C₆-C₁₄-Alylreste sind beispielsweise Phenyl, o-, m-, p-Tolyl, Benzyl, 2,3-, 2,4-, 2,5-, 2,6-, 3,4-, 3,5-Xylyl, Mesityl oder Naphthyl und C₁-C₄-Hydroxyalkylreste sind beispielsweise Hydroxymethyl, 1-Hydroxyethyl, 2-Hydroxyethyl, 1-Hydroxypropyl, 2-Hydroxypropyl, 3-Hydroxypropyl, 1-Hydroxybutyl, 2-Hydroxybutyl, 3-Hydroxybutyl, 4-Hydroxybutyl. Die vorangehende Aufzählung dient der beispielhaften Erläuterung der Erfindung und ist nicht als abschließend zu betrachten.

Als Substituenten der oben genannten Reste kommen zahlreiche organische Gruppen in Frage, beispielsweise Halogen-, insbesondere Fluor, Chlor oder Brom, sowie Ether-, Thioether-, Disulfid-, Sulfoxid-, Sulfon-, Aldehyd-, Keto-, Carbonsäureester-, Sulfonsäureester-, Carbonat-, Cyano-, Alkylsilan- und Alkoxysilangruppen und/oder Carboxylamidgruppen.

Als Dehydrierungsmittel im erfindungsgemäßen Verfahren eignen sich solche, die zur Dehydrierung von 2,3-Dihydro-, 2,5-Dihydro- oder Tetrahydrothiophenen sowie deren Derivaten geeignet sind. Bevorzugt sind dies solche, die eine Dehydrierung der Verbindungen der allgemeinen Formel .(II) bewirken, jedoch keine anschließende oxidative Polymerisation der erhaltenen Verbindungen der allgemeinen Formel (I) unterstützen. Durch die Dehydrierung findet eine Aromatisierung der Dihydrothiophene bzw. Verbindungen der allgemeinen Formel (II) statt. Die Dehydrierung kann im Sinne der Erfindung auf unterschiedlichen Wegen erfolgen:
- durch Thiolierung und Eliminierung von Schwefelwasserstoff
- durch Hydridionentransfer
- durch Halogenierung mittels Halogenierungsreagenzien und Eliminierung von Halogenwasserstoff
- durch S-Oxidation mittels geeigneter Oxidationsmittel und Dehydratisierung.

Bevorzugt eingesetzte Dehydrierungsmittel im erfindungsgemäßen Verfahren sind Chinone, Schwefel, Brom, N-Brom- oder N-Chlorsuccinimid, Sulfurylchlorid, Wasserstoffperoxid oder hypervalente Iodverbindungen wie Iodosobenzol. Bei der Dehydrierung mit Chinonen findet eine direkte Hydrierung des eingesetzten Dehydrierungsmittels statt (Hydridionentransfer), bei Einsatz von beispielsweise Brom, N-Brom- oder N-Chlorsuccinimid oder Sulfurylchlorid findet eine Halogenierung und nachfolgende Eliminierung von Halogenwasserstoff statt, Wasserstoffperoxid oder Iodosobenzol beispielsweise bewirken eine S-Oxidation gefolgt von einer Dehydratisierung und Schwefel bewirkt eine Thiolierung gefolgt von einer Eliminierung von Schwefelwasserstoff. Derartige Reaktionen sind beschrieben in Houben-Weyl, Methoden der Organischen Chemie (4. Aufl., Band E 6a (Hetarene I, Teil 1), Hrsg. R. P. Kreher, Georg Thieme-Verl, Stuttgart-New York 1994, im Kapitel "Thiophene" (Autor: W.-D. Rudorf) auf den Seiten 370 bis 388 in den Abschnitten 3. 3. bis 3. 8.).

Besonders bevorzugte Dehydrierungsmittel für den Einsatz im erfindungsgemäßen Verfahren sind Chinone, darunter ganz besonders bevorzugt 2,3,5,6-Tetrachlor-1,4-benzochinon (Chloranil) oder 2,3-Dichlor-5,6-dicyan-1,4-benzochinon.

Das erfindungsgemäße Verfahren wird prinzipiell so durchgeführt, dass die Verbindungen der allgemeinen Formel (II) mit mindestens äquimolaren oder überschüssigen Mengen an Dehydrierungsmittel bei geeigneter Temperatur und gegebenenfalls in einem organischen Lösemittel für 0,5 bis 48, bevorzugt 2 bis 24 h umgesetzt werden. Bevorzugt werden auf 1 Mol der Verbindung der allgemeinen Formel (II) 1 bis 2 Mol, besonders bevorzugt 1 bis 1,2 Mol an Dehdrierungsmittel eingesetzt, wobei in vielen Fällen ein genau äquimolares Verhältnis zwischen der Verbindung der allgemeinen Formel (II) und Dehydrierungsmittel ausreichend ist. Gegebenenfalls ausgefallener Feststoff wird abfiltriert und durch Aufschlämmen und anschließendes Filtrieren von unlöslichen Bestandteilen befreit. Zur Aufschlämmung eignet sich beispielsweise ein Gemisch aus organischem Lösemittel und wässriger Alkalilauge, wie z.B. 4%iger wässriger KOH-Lösung. Nach basischem Waschen und anschließendern Neutralwaschen mit Wasser sowohl dieser erhaltenen Mutierlauge als auch der Mutterlauge, die nach Filtration der Reaktionsmischung erhalten wurde, werden die Mutterlaugen eingeengt und im Vakuum getrocknet. Zum basischen Waschen eignen sich ebenfalls wässriger Alkalilaugen, wie z.B. 4%ige wässrige KOH-Lösung. Das erhaltene Rohprodukt kann nach gängigen Methoden, wie beispielsweise Säulenchromatographie oder durch Umkristallisation, aufgereinigt werden, weiterhin eignet sich hierzu auch die Entfernung stark färbende Verunreinigungen mit Bleicherde (Montmorillonit), z. B. Tonsil^{®}.

Geeignete Reaktionstemperaturen für die Durchführung des erfindungsgemäßen Verfahrens sind z. B. 20 bis 200°C, bevorzugt 100 bis 170°C, wobei es vorteilhaft sein kann, unter Rückfluss des verwendeten Lösemittels zu arbeiten. Grundsätzlich sind zudem auch niedrigere Temperaturen von 20°C bis hin zu -78°C geeignet, jedoch aufgrund der aufwändigen externen Kühlung bzw. Verwendung von kältemischungen von untergeordneter Bedeutung. Geeignete Lösernittel sind z. B. gegebenenfalls halogenierte aromatische Kohlenwasserstoffe, wie Toluol, Xylol, Chlorbenzol oder o-Dichlorbenzol. Es können allerdings auch weitere unter den Reaktionsbedingungen inerte, vorzugsweise organische, Lösemittel gegebenenfalls im Gemisch mit Wasser verwendet werden.

### Beispiele

### Allgemeine Vorschrift zur Synthese der Verbindungen der allgemeinen Formel (II):

Beispielhaft kann die Synthese der Verbindungen der allgemeinen Formel (II) so erfolgen, dass eine Lösung des 3,4-Alkylendioxythiophens in Methylenchlorid mit 0,5 bis 80 Gew-% einer Protonensäure, z.B. p-Toluolsulfonsäure oder Trifluoressigsäure, oder 0,01 bis 50 Gew.-% einer Lewissäure, z.B. Bortrifluorid in Form des Etherats oder eines anderen BF₃-Komplexes, als Katalysator bei Temperaturen von 0°C bis 40°C gegebenenfalls unter N₂-Atmosphäre umgesetzt wird. Die Umsetzung kann mehrere Stunden durchgeführt oder ¹H-NMR-spektroskopisch verfolgt und zum geeigneten Zeitpunkt durch Zugabe von Basen (z.B. wässrige Na₂CO₃-Lösung) -oder Alkoholen (z.B. Ethanol) abgebrochen werden. Die erhaltenen Verbindungen der allgemeinen Formel (II) können anschließend säulenchromatographisch, z.B. an Silicagel mit Methylenchlorid als Laufmittel, aufgereinigt werden.

### Herstellung von 2,2',3,3',5,7-Hexahydro-5,5'-bithieno[3,4-b][1,4]dioxin (II-a) (EDT-Dimer) durch Trifluoressigsäure-Katalyse

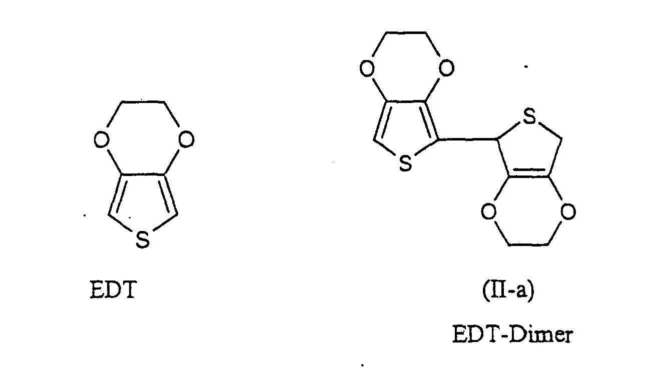

Wie in der allgemeinen Vorschrift beschrieben, wurden zu einer Lösung von 42,6 g (0,3 Mol) 3,4-Ethytendioxythiophen (EDT) in 70 ml Methylenchlorid 2,13 g Trifluoressigsäure in 90 ml Methylenchlorid bei 18°C zugegeben. Das Reaktionsgemisch wurde 48 h unter N₂-Atmosphäre bei 18°C gerührt. Danach wurde die Reaktion durch intensives Rühren mit 150 ml 5%iger wässriger Na₂CO₃-Lösung gestoppt. Die organische Phase wurde abgetrennt, mit Wasser neutral gewaschen und am Rotationsverdampfer bei 20 mbar vom Lösungsmittel befreit. Der Rückstand (42,2 g) wurde an Silicagel säulenchromatographisch mit Methylenchlorid als Laufmittel aufgetrennt.
- Friktion 1:: 21 g EDT
- Fraktion 2:: 11 g EDT-Dimer, identifiziert mittels ¹H-NMR in CDCl₃. Beige Kristalle,
- Schmp.:: 115-119 °C.
- Fraktion 3:: 1 g EDT-Trimer als Isomerengemisch, identifiziert mittels ¹H-NMR in CDCl₃. Beige Kristalle.

Das Produkt aus Fraktion 2 wird in den erfindungsgemäßen Beispielen 1 und 2 eingesetzt.

### Herstellung von Dimethyl-2,2',3,3',5,7-hexahydro-5,5'-bithieno[3,4-b][1,4]dioxin (II-b) (Methyl-EDT-Dimer) durch BF₃-Katalyse

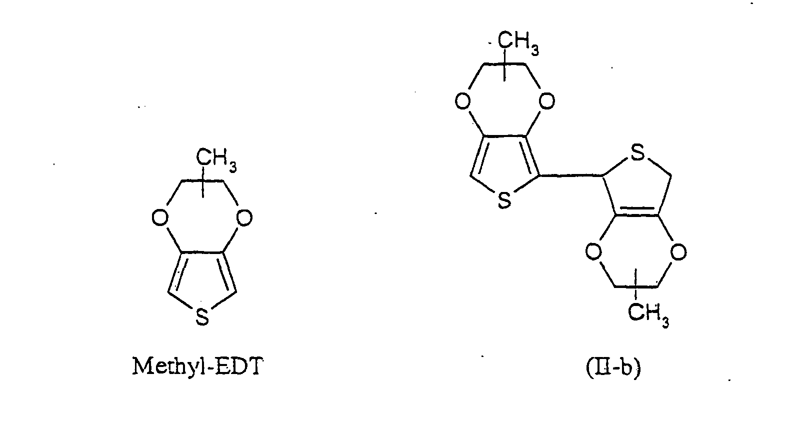

Wie in der allgemeinen Vorschrift beschrieben, wurden zu einer Lösung von 6,25 g (40 mMol) 2-Methyl-23-dihydrothieno[3,4-b][1,4]dioxin (Methyl-EDT) in 30 ml Methylenchlorid 0,24 ml Bortrifluorid-Etherat (entsprechend 269 mg = 1,89 mMol) in 40 ml Methylenchlorid bei 23°C zugegeben. Das Reaktionsgemisch wurde 7 h unter N₂-Atmosphäre bei 23°C gerührt. Danach wurde die Reaktion durch Zugabe von 20 ml Ethanol gestoppt. Die organische Phase wurde mit Wasser neutral gewaschen und am Rotationsverdampfer bei 20 mbar vom Lösungsmittel befreit. Der Rückstand wurde an Silicagel säulenchromatographisch mit Methylenchlorid als Laufmittel aufgetrennt. Ausbeute: 1,3 g Methyl-EDT-Dimer (II-b) (20,8 % d. Th.).

### Herstellung von [(Hydroxymethyl)-2,2',3,3',5,7-hexahydro-5,5'-bithieno[3,4-b][1,4]dioxinyl]methanol (II-c) (Hydroxymethyl-EDT-Dimer)

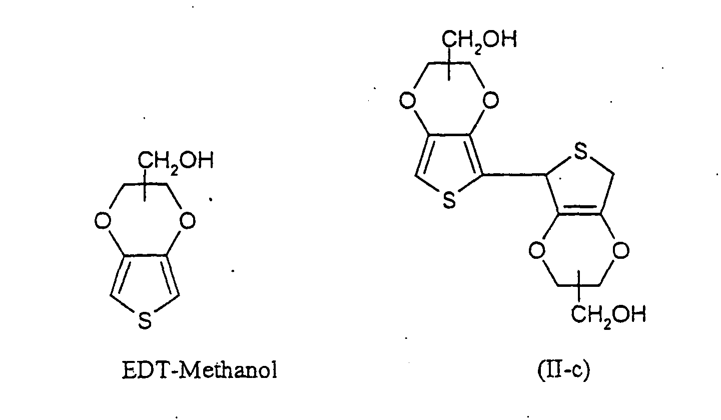

Wie in der allgemeinen Vorschrift beschrieben, wurden zu einer Lösung von 6,89 g (40 m Mol) 2,3-Dihydrothieno[3,4-b][1,4]dioxin-2-ylmethanol (Hydroxymethyl-EDT, "EDT-Methanol") in 90 ml Methylenchlorid 0,24 ml Bortrifluorid-Etherat (entsprechend 269 mg = 1,89 mMol) in 120 ml Methylenchlorid bei 0°C zugegeben. Das Reaktionsgemisch wurde 4 h unter N₂-Atmosphäre bei 0°C gerührt. Danach wurde die Reaktion durch Zugabe von einem Gemisch aus 60 ml Ethanol/120 ml Wasser gestoppt. Die organische Phase wurde abgetrennt und am Rotationsverdampfer bei 20 mbar vom Lösungsmittel befreit. Der Rückstand wurde an Silicagel säulenchromatographisch mit Methylenchlorid als Laufmittel aufgetrennt. Ausbeute 2,0 g Hydroxymethyl-EDT-Dimer (II-c) (29 % d. Th.)

### Beispiel 11: Herstellung von 2,2'-Di(3,4-ethylendioxythiophen) (I-a) (erfindungsgemäß)

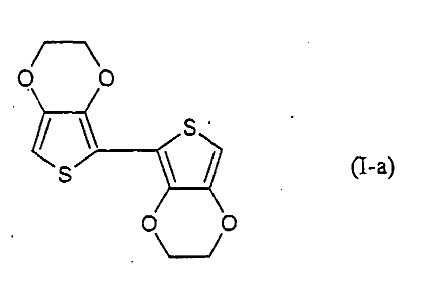

28,64 g (0,1 Mol) EDT-Dimer (II-a) wurden in 500 ml Xylol gelöst. Zu dieser Lösung wurden 24,59 g (0,1 Mol) Chloranil zugegeben. Das Gemisch wurde 10 h bei 140°C zum Rückfluss erhitzt. Nach Abkühlen wurden die ausgefallenen Kristalle abgesaugt und sowohl die Kristalle als auch die Mutterlauge weiter aufgearbeitet.

Die Mutterlauge wurde 4 mal mit je 50 ml 4 %iger Kalilauge gewaschen und zum Schluss mit Wasser neutral gewaschen. Die Lösung wurde eingedampft und im Hochvakuum getrocknet; man erhielt 15,8 g Rohprodukt als grüne Kristalle (Fraktion A).

Die abgesaugten Kristalle wurden mit 150 ml Xylol und 100 ml 4 %iger Kalilauge aufgeschlämmt und die unlöslichen Anteile (5,5 g) abfiltriert. Die Mutterlauge wurde wie die obige Mutterlauge weiter aufgearbeitet und ergab nach Eindampfen weitere 6,4 g Rohprodukt als grüne Kristalle (Fraktion B).

Die Rohprodukte (Fraktion A und B) wurden in 370 ml Methylenchlorid gelöst und mit 21 g Tonsil^{®} (Bleicherde vom Montmorillonit-Typ, Hersteller: Süd-Chemie, München) aufgeschlämmt. Nach Entfärbung der Lösung wurde das Tonsil^{®} abfiltriert und das Methylenchlorid am Rotationsverdampfer entfernt.

Es werden 18 g (63 % der Theorie) 2,2'-Di(3,4-ethylendioxythiophen) (I-a) als hellgelbe Kristalle isoliert. Schmp.: 204 - 205 °C.
¹H-NMR-Spektrum (400 MHz, CDCl₃, ppm): 4,24 (4H, m), 4,31 (4H, m), 6,27 (2H, s).

Aus den 5,5 g unlöslichen Anteilen wurden durch Aufschlämmen mit Tonsil^{®} in Methylenchlorid nach der gleichen Methodik weitere 1,5 g 2,2'-Di(3,4-ethylendioxythiophen) gewonnen. Gesamtausbeute: 69 % der Theorie.

### Beispiel 2: Herstellung von 2,2'-Di(3,4-ethylendioxythiophen) (I-a) mit 2,3-Dichlor-5,6-dicyan-p-benzochinon

8,53 g (30 mmol) EDT-Dimer (II-a) wurden in 150 ml Xylol gelöst. Zu dieser Lösung wurden 7,15 g (31,5 mmol) 2,3-Dichlor-5,6-dicyan-p-benzochinon zuge.-geben. Das Gemisch wurde 24 h bei 140 °C zum Rückfluss erhitzt. Nach Abkühlen wurde mit 4 %iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 3,68 g (43,5 % d. Th.) Rohprodukt als graubraunen Rückstand. Dieses Rohprodukt wurde chromatographisch über eine Silicagel-Säule gereinigt, Laufmittel Methylenchlorid.

Es wurden 1,32 g (15,6 % der Theorie) reines 2,2'-Di(3,4-ethylendioxythiophen) (I-a) isolier. Schmp.: 207 - 209 °C.

Das ¹H-NMR-Spektrum (400 MHz, CDCl₃) ist identisch mit dem in Beispiel 1 angegebenen.

### Beispiel 3: Herstellung von Dimethyl-2,2',3,3'-tetrahydro-5,5'-bithieno[3,4-b][1,4]dioxin (I-b)

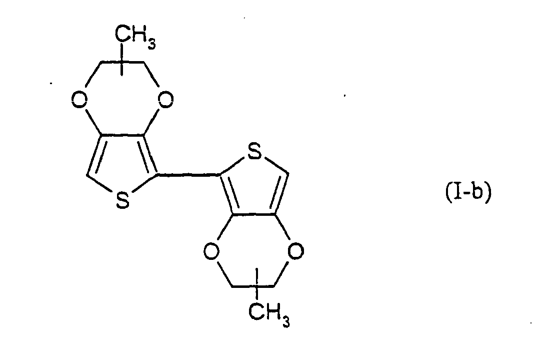

0,85 g (2,7 mmol) Methyl-EDT-Dimer (II-b) wurden in 50 ml Xylol gelöst. Zu dieser Lösung wurden 0,668 g (2,7 mmol) Chloranil zugegeben. Das Gemisch wurde 14 h bei 140°C zum Rückfluss erhitzt. Nach Abkühlen wurde mit Methylenchlorid verdünnt und mit 4%iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 0,48 g (56,7 % d. Th.) rohes (I-b) als Rückstand. Das Product wurde säulenchromatographisch an Kieselgel, Laufmittel CH₂Cl₂, gereinigt. Reinausbeute 0,38 g (44,8 % d. Th.).

¹H-NMR-Spektrum (400 MHz, CDCl₂, ppm): 6,24, 6,235, 6,23 (3 s, zus, 2H); 4,39 (weiter aufgespaltenes q, ³*J* = 6,56 Hz) und 4,32 (weiter aufgespaltenes q, ³*J* = 6,56 Hz, beide q zus. 2H); 4,28 (dd, ²*J* = 11,60 Hz, ³*J* = 2,04 Hz) und 4,15 (dd, ²*J* = 11,60 Hz, ³ *J* = 2,04 Hz, beide dd zus. 2 H); 3,91 (dd, ²*J* = 11,60 Hz, ³J = 8,56 Hz) und 3,865 (dd, ²*J* = 11,60 Hz, ³*J* = 8,56 Hz, beide dd zus. 2H); 1,42 (d, ³*J* = 6,56 Hz) und 1,355 (d, ³*J* = 6,56 Hz, beide d zus. 6H). Das NMR-Spektrum legt das Vorliegen eines Produktgemisches aus folgenden Komponenten nahe:

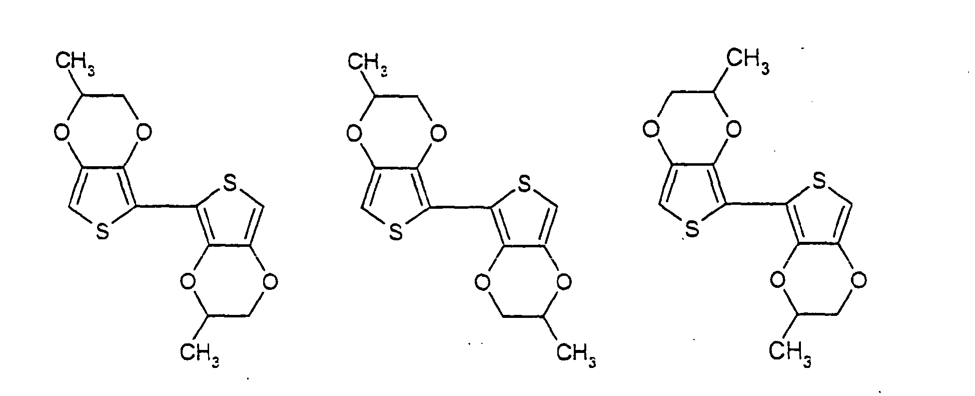

### Beispiel 4: Herstellung von (Hydroxymethyl)-2,2',3,3'-tetrahydro-5,5'-bithieno[3,4-b][1,4]dioxinyl]methanol (I-c)

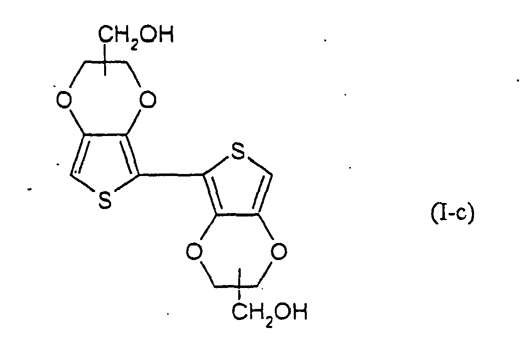

2,4 (7 mmol) Hydroxymethyl-EDT-Dimer (II-c) wurden in 50 ml Xylol gelöst. Zu dieser Lösung wurden 1,713 g (7 mmol) Chloranil zugegeben. Das Gemisch wurde 24 h bei 140°C zum Rückfluss erhitzt. Nach Abkühlen würde mit 4 %iger Kalilauge ausgeschüttelt, bis die Kalilauge farblos blieb. Zum Schluss wurde mit Wasser neutral gewaschen. Die Lösung wurde eingedampft und im Hochvakuum getrocknet; man erhielt 0,2 g (8,3 % d. Th.) (I-c) als Rückstand.

¹H-NMR-Spektrum (400 MHz, CDCl₃, ppm): 6,285; 6,275; 6,27; 6,26 (4 s, zus. 2H), 4,21 - 3,73 (mehrere m, zus. 6H). Das NMR-Spektrum legt das Vorliegen eines Produktgemisches aus folgenden Komponenten nahe:

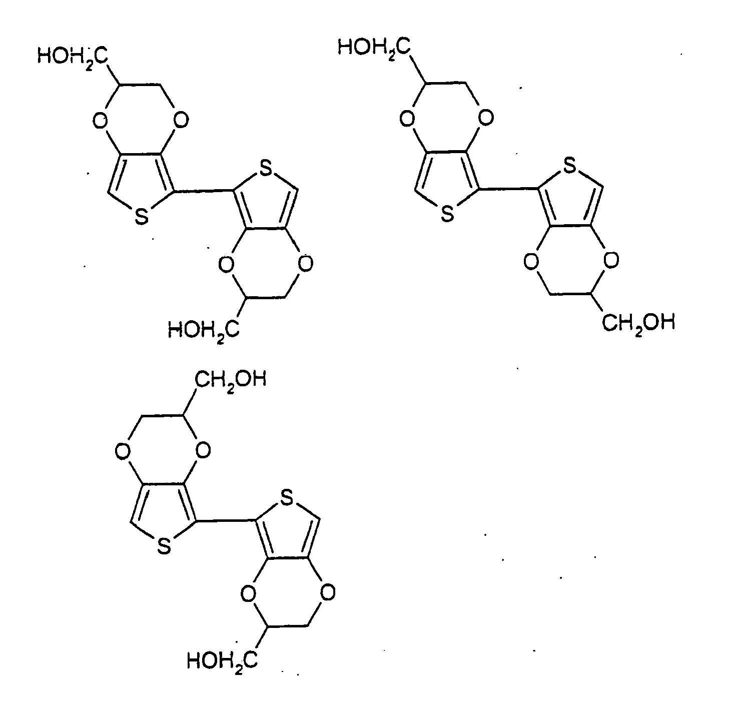

## Patentansprüche

1. Verfahren zur Herstellung von Verbindungen der allgemeinen Formel (I), 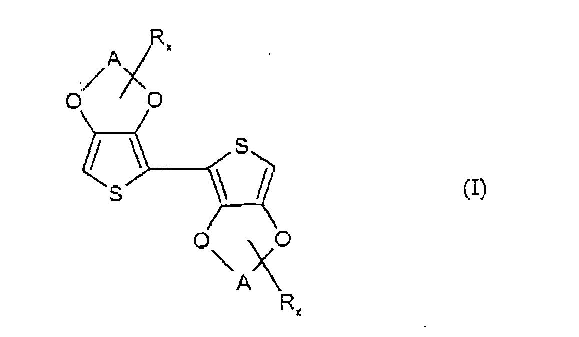 worin
A für einen C₂-C₄-Alkylenrest steht,
R für einen oder mehrere, gleiche oder verschiedene Lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenen- falls substituierte(n) C₆-C₁₄-Arylrest(e), gegebenenfalls substi- tuierte(n) C₁-C₄-Hydroxyalkylrest(e) oder einen oder mehrere Hy- droxylrest(e) steht,
x für eine ganze. Zahl von 0 bis 8 steht,
**dadurch gekennzeichnet dass** Verbindungen der allgemeinen Formel (II), 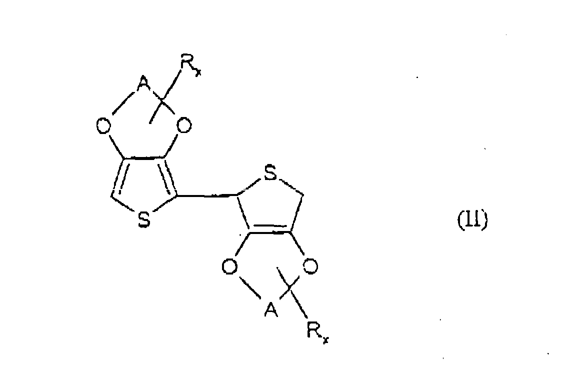 worin
A, R und x die für die Verbindungen der allgemeinen Formel (1) genannte Bedeutung haben,
mit einem Dehydrierungsmittel umgesetzt werden.

2. Verfahren gemäß Anspruch 1, **dadurch gekennzeichnet, dass**
A für einen Ethylenrest steht,
R für einen oder mehrere, gleiche oder verschiedene lineare(n) oder verzweigte(n), gegebenenfalls substituierte(n) C₁-C₁₈-Alkylrest(e)*,* gegebenenfalls substituierte(n) C₅-C₁₂-Cycloalkylrest(e), gegebenen- falls substituierte(n) C₆-C₁₄-Arylrest(e), gegebenenfalls substi- tuierte(n) C₁-C₂-Hydroxyalkylrest(e) oder einen oder mehrere Hydroxylrest(e) steht,
x für eine ganze Zahl von 0 bis 4 steht.

3. Verfahren gemäß Anspruch 1 oder 2, **dadurch gekennzeichnet, dass**
A für einen Ethylenrest steht,
R für Methyl, Ethyl, n-Propyl, n-Butyl, n-Hexyl, n-Octyl, n-Decyl, n- Dodecyl, n-Tetradecyl oder Hydroxymethyl steht,
x für 0 oder 1 steht.

4. Verfahren gemäß wenigstens einem der Ansprüche 1 bis 3 zur Herstellung der
Verbindung der Formel (I-a) 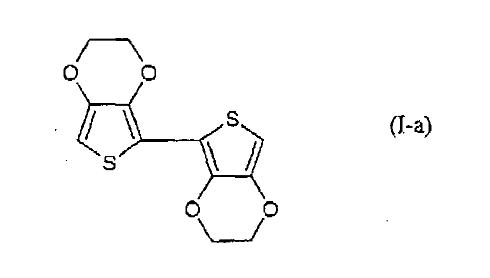 **dadurch gekennzeichnet, dass** die Verbindungen der Formel (II-a) 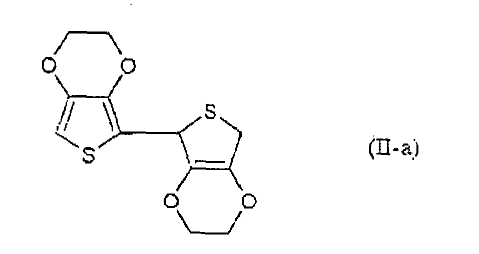 mit einem Dehydrierungsmittel umgesetzt werden.

5. Verfahren gemäß wenigstens einem der Ansprüche 1 bis 4, **dadurch gekennzeichnet, dass** das Dehydrierungsmittel ausgewählt ist aus der Gruppe der Chinone. Schwefel, Brom, N-Chlor- oder N-Bromsuccinimid, Sulfurylchlorid, Wasserstoffperoxid oder Iodosobenzol

6. Verfahren gemäß wenigstens einem der Ansprüche 1 bis 5, **dadurch gekennzeichnet, dass** das Dehydrierungsmittel ein Chinon ist.

7. Verfahren gemäB wenigstens einem der Ansprücbe 1 bis 6, **dadurch gekennzeichnet, dass** das Dehydrierungsmittel im erfindungsgemäßen Verfahren 2,3,5,6-Tetrachlor-1,4-benzochinon (Chloranil) oder 2,3-Dichlor-5,6-dicyan-1,4-benzochinon ist.

## Claims

1. Process for preparing compounds of the general formula (I) 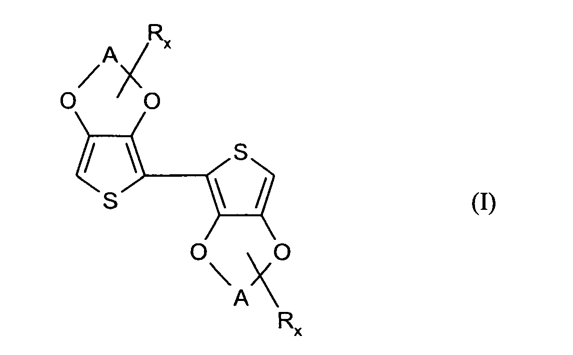 where
A is a C₂-C₄-alkylene radical,
R is one or more, identical or different, linear or branched, optionally substituted C₁-C₁₈-alkyl radical(s), optionally substituted C₅-C₁₂-cycloalkyl radical(s), op- tionally substituted C₆-C₁₄-aryl radical(s), optionally substituted C₁-C₄- hydroxyalkyl radical(s) or one or more hydroxyl radical(s),
x is an integer from 0 to 8,
**characterized in that** compounds of the general formula (II) 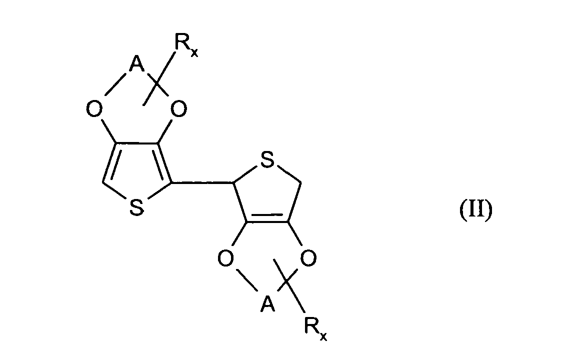 where
A, R and x are each as defined for the compounds of the general formula (I)
are reacted with a dehydrogenating agent.

2. Process according to Claim 1, **characterized in that**
A is an ethylene radical,
R is one or more, identical or different, linear or branched, optionally substituted C₁-C₁₈-alkyl radical(s), optionally substituted C₅-C₁₂-cycloalkyl radical(s), op- tionally substituted C₆-C₁₄-aryl radical(s), optionally substituted C₁-C₄- hydroxyalkyl radical(s) or one or more hydroxyl radical(s),
x is an integer from 0 to 4.

3. Process according to Claim 1 or 2, **characterized in that**
A is an ethylene radical,
R is methyl, ethyl, n-propyl, n-butyl, n-hexyl, n-octyl, n-decyl, n-dodecyl, n- tetradecyl or hydroxymethyl,
x is 0 or 1.

4. Process according to at least one of Claims 1 to 3 for preparing the compound of the
formula (I-a) 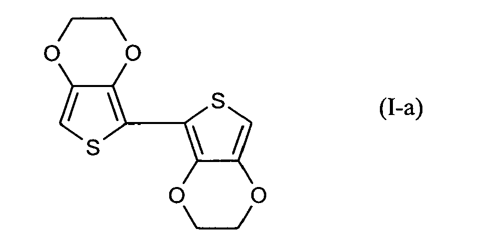 **characterized in that** the compounds of the formula (II-a) 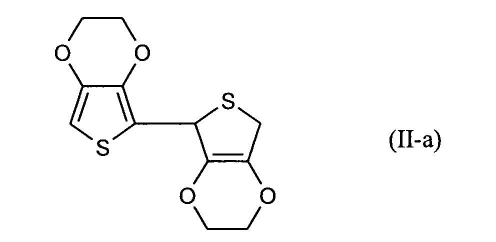 are reacted with a dehydrogenating agent.

5. Process according to at least one of Claims 1 to 4, **characterized in that** the dehydrogenating agent is selected from the group of the quinones, sulphur, bromine, N-chloro- or N-bromosuccinimide, sulphuryl chloride, hydrogen peroxide or iodosobenzene.

6. Process according to at least one of Claims 1 to 5, **characterized in that** the dehydrogenating agent is a quinone.

7. Process according to at least one of Claims 1 to 6, **characterized in that** the dehydrogenating agent in the process according to the invention is 2,3,5,6-tetrachloro-1,4-benzoquinone (chloranil) or 2,3-dichloro-5,6-dicyano-1,4-benzoquinone.

## Revendications

1. Procédé de préparation de composés de formule générale (I), 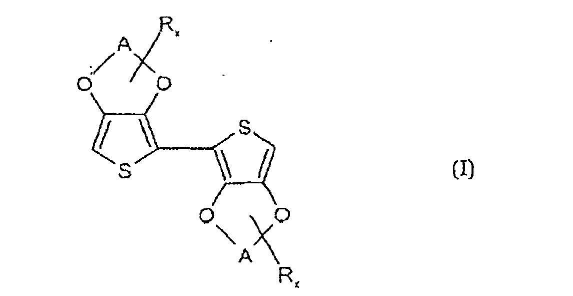 dans laquelle
A représente un radical alkylène en C₂-C₄,
R représente un ou plusieurs radicaux identiques ou différents, linéaires ou ramifiés, alkyles en C₁-C₁₈ éventuellement substitués, cycloalkyles en C₅-C₁₂ éventuellement substitués, aryles en C₆-C₁₄ éventuellement substitués, hydroxyalkyles en C₁-C₄ éventuellement substitués, ou un ou plusieurs radicaux hydroxyles,
x représente un nombre entier de 0 à 8,
**caractérisé en ce que** l'on fait réagir des composés de formule générale (II), 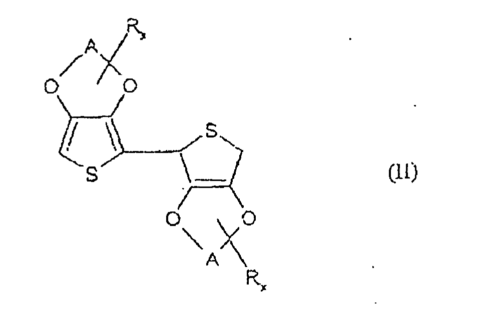 dans laquelle
A, R et X ont la signification indiquée pour les composés de formule générale (I),
avec un agent déshydrogénant.

2. Procédé selon la revendication 1, **caractérisé en ce que**
A représente un radical éthylène,
R représente un ou plusieurs radicaux identiques ou différents, linéaires ou ramifiés, alkyles en C₁-C₁₈ éventuellement substitués, cycloalkyles en C₅-C₁₂ éventuellement substitués, aryles en C₆-C₁₄ éventuellement substitués, hydroxyalkyles en C₁-C₄ éventuellement substitués, ou un ou plusieurs radicaux hydroxyles,
x représente un nombre entier de 0 à 4.

3. Procédé selon la revendication 1 ou 2, **caractérisé en ce que**
A représente un radical éthylène,
R représente un radical méthyle, éthyle, n-propyle, n-butyle, n-hexyle, n- octyle, n-décyle, n-dodécyle, n-tétradécyle, ou hydroxyméthyle,
x représente 0 ou 1.

4. Procédé selon au moins l'une des revendications 1 à 3 pour la préparation du composé de formule (I-a) 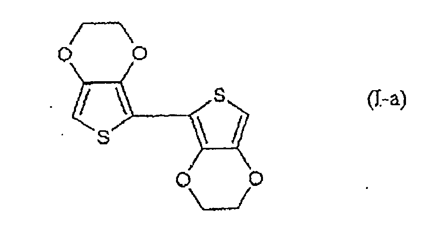 **caractérisé en ce que** l'on fait réagir les composés de formule (II-a) 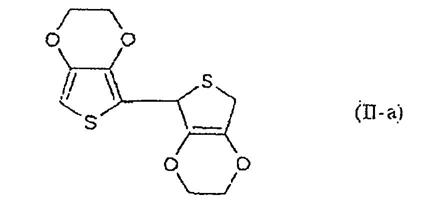 avec un agent déshydrogénant.

5. Procédé selon au moins l'une des revendications 1 à 4, **caractérisé en ce que** l'agent déshydrogénant est choisi dans le groupe des quinones, du soufre, du brome, du N-chloro- ou N-bromosuccinimide, du chlorure de sulfuryle, du peroxyde d'hydrogène ou de l'iodosobenzène.

6. Procédé selon au moins l'une des revendications 1 à 5, **caractérisé en ce que** l'agent déshydrogénant est une quinone.

7. Procédé selon au moins l'une des revendications 1 à 6, **caractérisé en ce que** l'agent déshydrogénant dans le procédé selon l'invention est la 2,3,5,6-tétrachloro-1,4-benzoquinone (chloranile) ou la 2,3-dichloro-5,6-dicyano-1,4-benzoquinone.
